# EUROPEAN PATENT SPECIFICATION

(11) **EP 3 266 477 B1**
(45) Date of publication and mention of the grant of the patent: **24.02.2021**
(21) Application number: 16758991.0
(22) Date of filing: 03.03.2016
(51) Int. Cl.: A61M 1/36, B01D 19/00

(54) **AIR TRAP CHAMBER**
LUFTFANGKAMMER
CHAMBRE DE TRAPPAGE

(30) Priority: 03.03.2015 JP 2015041227
(43) Date of publication of application: 10.01.2018
(73) Proprietor: Nikkiso Company Limited, Tokyo 150-6022 (JP)
(72) Inventor: SUGIYAMA, Hironobu, Makinohara-shi Shizuoka 421-0496 (JP); ONO, Kazuhide, Makinohara-shi Shizuoka 421-0496 (JP); FUNAMURA, Shigeaki, Makinohara-shi Shizuoka 421-0496 (JP)
(74) Representative: von Hirschhausen, Helge
(86) International application number: PCT/JP2016/056548
(87) International publication number: WO 2016/140299

(56) References cited:
- JP-A- H06 142 192
- US-A- 4 102 655
- US-A- 4 344 777
- US-A- 5 674 199
- US-A- 5 931 990
- US-A1- 2007 269 340
- US-A1- 2009 084 721
- US-A1- 2010 292 627
- US-B1- 6 176 903

## Description

### Technical Field

The present invention relates to an air-trap chamber connected to a blood flow route in which blood is made to flow, the air-trap chamber including a chamber portion capable of storing a predetermined volume of blood, a blood inlet from which blood is allowed to be introduced into the chamber portion, and a blood outlet from which the blood in the chamber portion is allowed to be discharged, the air-trap chamber being configured to collect and remove air bubbles contained in the blood stored in the chamber portion.

### Background Art

In general, a blood purification apparatus for performing dialysis treatment includes an arterial blood circuit and a venous blood circuit that form a blood circuit for extracorporeally circulating the blood of a patient, a blood purifier for purifying the blood that is under extracorporeal circulation through the blood circuit, and an apparatus body in which various treatment devices such as a blood pump for performing blood purification treatment with the blood circuit and the blood purifier are provided. An arterial puncture needle and a venous puncture needle are attachable to the distal ends of the arterial blood circuit and the venous blood circuit, respectively.

When the blood pump is activated after the arterial puncture needle and the venous puncture needle are stuck into the patient, blood of the patient flows through the blood circuit (the arterial blood circuit and the venous blood circuit), whereby the blood is purified by the blood purifier. Typically, the blood circuit is provided with air-trap chambers for collecting and removing air bubbles contained in the blood flowing through the blood circuit.

Typically, the air-trap chamber includes a chamber portion capable of storing a predetermined volume of blood, a blood inlet from which the blood in the blood circuit is allowed to be introduced into the chamber portion, and a blood outlet from which the blood in the chamber portion is allowed to be discharged into the blood circuit. When the blood introduced from the blood inlet flows down onto the surface of the blood stored in the chamber portion, bubbles may be generated by the blood gaining momentum while flowing down. To assuredly collect air bubbles generated in such a situation, the capacity of the chamber portion needs to be increased. To address such a problem, some proposals have already been made regarding air-trap chambers each having a blood inlet with a mouth positioned below the surface of liquid stored in a chamber portion (see PTL 1, for example).

### Citation List

### Patent Literature

PTL 1: JP2014-506157 (a Published Japanese Translation of a PCT Application)
PTL 2: US5674199A1.
PTL 3: US2009/0084721.

### Summary of Invention

### Technical Problem

In each of the proposed air-trap chambers, blood introduced from the blood inlet does not flow down onto the surface of the blood stored in the chamber portion. Therefore, the foaming that may be caused by the blood gaining momentum while flowing down can be prevented. However, if the blood introduced from the blood inlet flows at a high speed, the blood may be discharged from the blood outlet with some air bubbles remaining in it. Hence, the capacity of the chamber portion needs to be increased.

The present invention has been conceived in view of the above circumstances and provides an air-trap chamber with which air bubbles contained in blood can be removed to a higher extent and more assuredly and whose chamber portion can therefore have a reduced capacity.

### Solution to Problem

The scope of the present invention is defined by the appended claims.

### Advantageous Effects of Invention

According to the invention of Claim 1, the guiding member that allows the predetermined liquid introduced from the liquid inlet to flow upward and then to flow downward into the chamber portion is provided. Therefore, air bubbles contained in the predetermined liquid can be removed to a higher extent and more assuredly. Consequently, the capacity of the chamber portion can be reduced.

According to the invention of Claim 2, the air-trap chamber further comprises the receiving surface that receives the predetermined liquid introduced from the liquid inlet. Furthermore, the predetermined liquid received by the receiving surface is allowed to flow downward along the guiding member into the chamber portion. Therefore, the momentum of the predetermined liquid introduced from the liquid inlet can be fully reduced when the predetermined liquid is received by the receiving surface. Accordingly, the foaming or the like in the chamber portion can be suppressed.

According to the invention of Claim 1, the guiding member is the wall part formed at the peripheral edge of the opening that allows the predetermined liquid to flow downward into the chamber portion. Therefore, air bubbles contained in the predetermined liquid can be removed to a higher extent and more assuredly with a simple configuration. Consequently, the capacity of the chamber portion can be reduced.

According to the invention of Claim 1, the air-trap chamber further comprises the air-layer portion on the upper side and the middle portion interposed between the air-layer portion and the chamber portion. Furthermore, the opening is provided in the middle portion and is provided at the position decentered with respect to the middle portion. Therefore, the following advantageous effects can be produced: (a) since the radial size can be reduced, the air-trap chamber can be manufactured in a compact size with a reduced amount of material; (b) since the air-trap chamber can be manufactured in a compact size, the priming volume (the amount of liquid to be charged) can be reduced; (c) since the air-trap chamber can be manufactured in a compact size, the time period (the retention time) for the substitution (turnover) of the predetermined liquid in the air-trap chamber can be reduced, suppressing, if the predetermined liquid is blood, the occurrence of blood coagulation and the generation of thrombus; and (d) since the liquid flow route (a side thereof opposite the ejection side) is narrowed gradually, the flow speed is increased and the time period (the retention time) for the substitution (turnover) of the predetermined liquid in the air-trap chamber can be reduced, suppressing, if the predetermined liquid is blood, the occurrence of blood coagulation and the generation of thrombus.

According to the invention of Claim 3, the wall part extends over the entirety of the peripheral edge of the opening and has, on the outer-peripheral side of the wall part, the reservoir part capable of storing the predetermined liquid introduced from the liquid inlet. Furthermore, the predetermined liquid stored in the reservoir part is allowed to flow over the top end of the wall part and to flow downward into the chamber portion. Therefore, air bubbles contained in the predetermined liquid can be removed while the predetermined liquid flows along the guiding member, and air bubbles contained in the predetermined liquid can also be removed while the predetermined liquid is stored in the reservoir part. Thus, air bubbles contained in the predetermined liquid can be removed to a much higher extent and much more assuredly. Consequently, the capacity of the chamber portion can further be reduced.

According to the invention of Claim 4, the air-trap chamber further comprises the projecting portion that projects upward from the position across the wall part from the receiving surface that receives the predetermined liquid introduced from the liquid inlet. Therefore, the overflow in a region where the predetermined liquid tends to be retained can be caused more proactively, and the retention of the predetermined liquid can be suppressed.

According to the invention of Claim 5, the air-trap chamber further comprises the cut part provided by cutting the region across the wall part from the receiving surface that receives the predetermined liquid introduced from the liquid inlet. Therefore, the overflow in the region where the predetermined liquid tends to be retained can be caused more proactively, and the retention of the predetermined liquid can be suppressed.

According to the invention of Claim 6 the air-trap chamber further comprises the ejection port that ejects, toward the reservoir part, the predetermined liquid introduced from the liquid inlet, and the cut provided in the ejection port. Therefore, the following advantageous effects can be produced: (a) since the radial size can be reduced, the air-trap chamber can be manufactured in a compact size with a reduced amount of material; (b) since the air-trap chamber can be manufactured in a compact size, the priming volume (the amount of liquid to be charged) can be reduced; (c) since the air-trap chamber can be manufactured in a compact size, the time period (the retention time) for the substitution of the predetermined liquid in the air-trap chamber can be reduced, suppressing, if the predetermined liquid is blood, the occurrence of blood coagulation and the generation of thrombus; and (d) since the liquid flow route (a side thereof opposite the ejection side) is narrowed gradually, the flow speed is increased and the time period (the retention time) for the substitution of the predetermined liquid in the air-trap chamber can be reduced, suppressing, if the predetermined liquid is blood, the occurrence of blood coagulation and the generation of thrombus.

According to the invention of Claim 7, the inner peripheral surface of the wall part and the inner peripheral surface of the chamber portion are continuous with each other, and the predetermined liquid that has flowed over the top end of the wall part is allowed to flow downward along the inner peripheral surface of the wall part and the inner peripheral surface of the chamber portion. Therefore, the foaming of the predetermined liquid whose surface is formed at the liquid level in the chamber portion can be suppressed more assuredly.

According to the invention of Claim the air-trap chamber further comprises the flow path having the ejection port from which the predetermined liquid introduced from the liquid inlet is ejected toward the reservoir part. Furthermore, the ejection port is positioned below the top end of the wall part. Therefore, the predetermined liquid introduced from the liquid inlet and ejected from the ejection port can be prevented from flowing with increased momentum toward the surface of the predetermined liquid stored in the reservoir part. Consequently, the foaming in the reservoir part can be suppressed more assuredly.

According to the invention of Claim 9 , a blood circuit comprising the air-trap chamber according to any of Claims 1 to 8 can be provided.

According to the invention of Claim 10 , a blood purification apparatus comprising the blood circuit according to Claim 9 can be provided.

### Brief Description of Drawings

[Fig. 1] Fig. 1 is a schematic diagram of a blood circuit to which an air-trap chamber according to each embodiment of the present invention is applied.
[Fig. 2] Fig. 2 includes a plan view and a front view of an air-trap chamber according to a first embodiment of the present invention.
[Fig. 3] Fig. 3 illustrates a section taken along line III-III illustrated in Fig. 2.
[Fig. 4] Fig. 4 illustrates a section taken along line IV-IV illustrated in Fig. 2.
[Fig. 5] Fig. 5 includes a plan view and a front view of an air-trap chamber according to a second embodiment of the present invention.
[Fig. 6] Fig. 6 illustrates a section taken along line VI-VI illustrated in Fig. 5.
[Fig. 7] Fig. 7 illustrates a section taken along line VII-VII illustrated in Fig. 5.
[Fig. 8] Fig. 8 illustrates a section taken along line VIII-VIII illustrated in Fig. 7.
[Fig. 9] Fig. 9 is a perspective view of the air-trap chamber and illustrates a wall part, a reservoir part, and so forth.
[Fig. 10] Fig. 10 is a sectional view of an air-trap chamber according to another embodiment of the present invention and illustrates a wall part, a reservoir part, and so forth.
[Fig. 11] Fig. 11 includes a plan view and a front view of an air-trap chamber according to a third embodiment of the present invention.
[Fig. 12] Fig. 12 illustrates a section taken along line XII-XII illustrated in Fig. 11.
[Fig. 13] Fig. 13 illustrates a section taken along line XIII-XIII illustrated in Fig. 11.
[Fig. 14] Fig. 14 illustrates a section taken along line XIV-XIV illustrated in Fig. 13.
[Fig. 15] Fig. 15 is a perspective view of the air-trap chamber and illustrates a wall part, a reservoir part, and so forth.
[Fig. 16] Fig. 16 is a partially broken perspective view of the air-trap-chamber.
[Fig. 17] Fig. 17 includes a plan view and a front view of an air-trap chamber according to a fourth embodiment of the present invention.
[Fig. 18] Fig. 18 illustrates a section taken along line XVIII-XVIII illustrated in Fig. 17.
[Fig. 19] Fig. 19 illustrates a section taken along line XIX-XIX illustrated in Fig. 17.
[Fig. 20] Fig. 20 is a perspective view of the air-trap chamber and illustrates a wall part, a reservoir part, and so forth.

### Description of Embodiments

Embodiments of the present invention will now be described specifically with reference to the drawings.

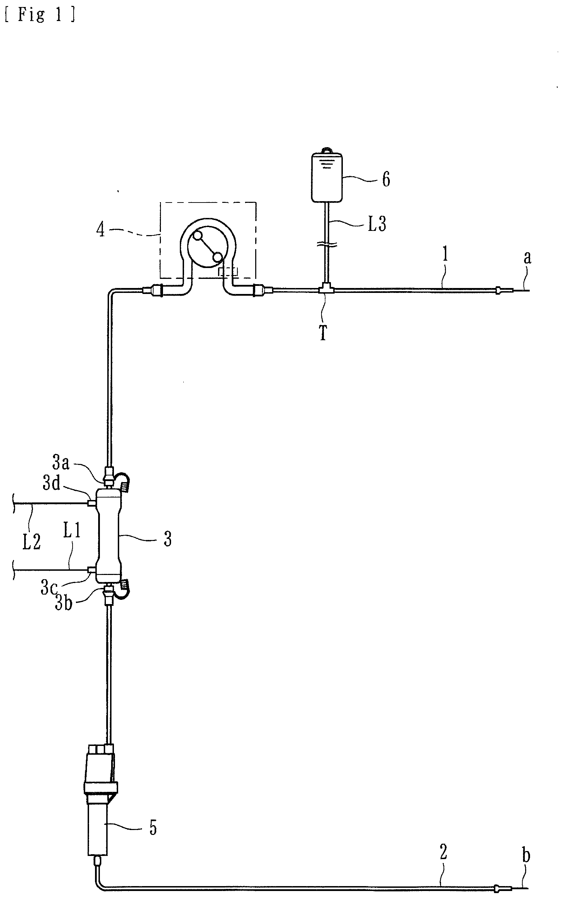

An air-trap chamber according to an embodiment is connected to a blood flow route (a liquid flow route) in which blood (a predetermined liquid) is made to flow, and is intended for collecting and removing air bubbles contained in the blood flowing in the blood flow route. The air-trap chamber is applied to a blood purification apparatus illustrated in Fig. 1. The blood purification apparatus is applied to a dialysis apparatus for purifying the blood of a patient while extracorporeally circulating the blood and includes a blood circuit (an arterial blood circuit 1 and a venous blood circuit 2), a dialyzer 3 as a blood purifier, and a blood pump 4.

The dialyzer 3 includes a housing that houses a plurality of hollow fibers each having very small holes (pores). The housing has a blood introduction port 3a, a blood discharge port 3b, a dialysate introduction port 3c, and a dialysate discharge port 3d. The blood circuit includes the arterial blood circuit 1 to the distal end of which an arterial puncture needle a is attachable, and the venous blood circuit 2 to the distal end of which a venous puncture needle b is attachable. The arterial blood circuit 1 is made of a flexible tube. The proximal end of the arterial blood circuit 1 is connected to the blood introduction port 3a of the dialyzer 3. The proximal end of the venous blood circuit 2 is connected to the blood discharge port 3b of the dialyzer 3.

In the dialysis apparatus, a dialysate introduction tube L1 through which dialysate is introduced into the dialyzer 3 and a dialysate discharge tube L2 through which the dialysate (drain liquid) is discharged from the dialyzer 3 are attachable to the dialyzer 3. The distal end of the dialysate introduction tube L1 is connected to the dialysate introduction port 3c of the dialyzer 3. The distal end of the dialysate discharge tube L2 is connected to the dialysate discharge port 3d of the dialyzer 3. Furthermore, a physiological-saline supply line L3 is connected to the arterial blood circuit 1 with a pipe tee T interposed therebetween. A container unit 6 called saline bag and being capable of containing a physiological saline solution is connected to the distal end of the physiological-saline supply line L3.

Furthermore, the arterial blood circuit 1 is provided at a halfway position thereof with the blood pump 4, which is a peristaltic pump. The blood pump 4 includes a pair of rollers and is capable of deliver liquid with the rotation of the rollers. When the blood pump 4 is activated, blood collected from the arterial puncture needle a is extracorporeally circulated through the arterial blood circuit 1 and the venous blood circuit 2, whereby the blood is purified by the dialyzer 3. Thus, blood purification treatment is performed. The venous blood circuit 2 is provided at a halfway position thereof with an air-trap chamber 5.

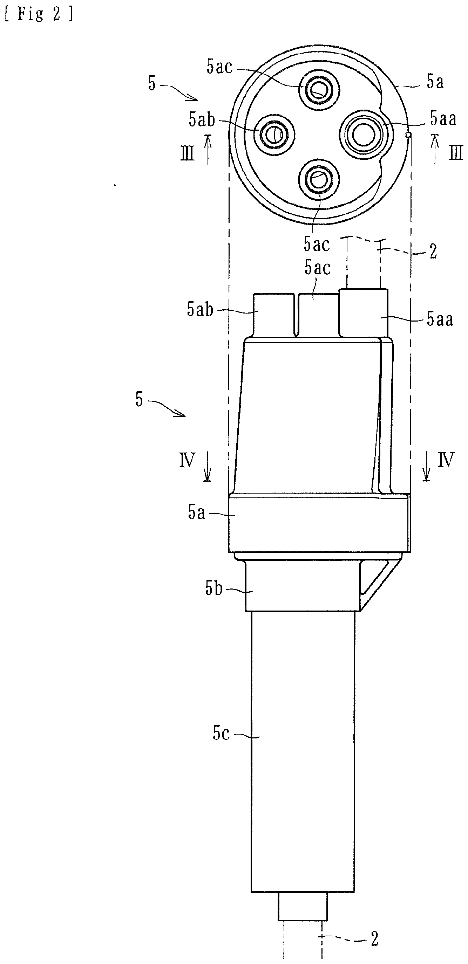

The air-trap chamber 5 according to the present embodiment is connected to a liquid flow route (the blood circuit) in which blood is made to flow. As illustrated in Figs. 2 to 4, the air-trap chamber 5 includes a chamber portion 5c capable of storing a predetermined volume of blood (a predetermined liquid), a blood inlet 5aa (a liquid inlet) from which blood in the blood flow route (the venous blood circuit 2) is allowed to be introduced into the chamber portion 5c, and a blood outlet 5ca (a liquid outlet) from which the blood in the chamber portion 5c is allowed to be discharged into the blood flow route (the venous blood circuit 2). The air-trap chamber 5 is intended for collecting and removing air bubbles contained in the blood stored in the chamber portion 5c.

More specifically, the air-trap chamber 5 according to the present embodiment basically includes an air-layer portion 5a on the upper side, the chamber portion 5c on the lower side, and a middle portion 5b interposed between the air-layer portion 5a and the chamber portion 5c. The air-layer portion 5a provides a storing space S1 having a predetermined capacity. The chamber portion 5c provides a storing space S2 having a predetermined capacity. The air-layer portion 5a has, at the top thereof, the blood inlet 5aa to which an upstream portion of the venous blood circuit 2 is connected, a connecting part 5ab to which an infusion line (not illustrated) or the like is connected, and a connecting part 5ac to which a monitor line (not illustrated) capable of detecting venous pressure is connected.

The blood inlet 5aa provided to the air-layer portion 5a is connected to a flow path 5ad extending downward in the air-layer portion 5a. An ejection port 5ae is provided at the distal end of the flow path 5ad and is positioned in the middle portion 5b. Hence, blood introduced from the blood inlet 5aa flows downward along the flow path 5ad, is ejected from the ejection port 5ae, flows through an opening K provided in the middle portion 5b, and flows downward into the chamber portion 5c.

The blood outlet 5ca to which a downstream portion of the venous blood circuit 2 is connected is provided at the bottom of the chamber portion 5c. Blood (or any other liquid such as a physiological saline solution) introduced into the chamber portion 5c from the blood inlet 5aa is stored in the storing space S2 provided in the chamber portion 5c, and is then discharged from the blood outlet 5ca. Hence, when blood or any other liquid is made to flow through the blood circuit, an air layer is formed in the storing space S1 of the air-layer portion 5a, whereas a liquid layer (a layer of blood or any other liquid) is formed in the storing space S2 of the chamber portion 5c. Thus, air bubbles contained in the liquid forming the liquid layer are collected into the air layer.

The middle portion 5b includes an outer peripheral wall 5ba, a bottom part 5bb, a wall part 7, and a reservoir part 8. The outer peripheral wall 5ba forms a substantially annular wall extending along the outline of the middle portion 5b. The opening K is provided on the inner side of the outer peripheral wall 5ba. The opening K corresponds to a region where the storing space S1 of the air-layer portion 5a and the storing space S2 of the chamber portion 5c communicate with each other. The wall part 7 projects from and extends over the entirety of the peripheral edge of the opening K.

The bottom part 5bb extends between the base end of the wall part 7 and the outer peripheral wall 5ba. The bottom part 5bb closes the gap between the storing space S1 of the air-layer portion 5a and the storing space S2 of the chamber portion 5c. The bottom part 5bb is a flat member extending horizontally over an area excluding the wall part 7 and the opening K in such a manner as to isolate the storing space S1 and the storing space S2 from each other. The bottom part 5bb forms the bottom surface of the reservoir part 8. That is, a space between the inner peripheral surface of the outer peripheral wall 5ba and an outer peripheral surface 7a of the wall part 7 serves as the reservoir part 8 having a bottom surface formed of the bottom part 5bb. Liquid such as blood introduced from the blood inlet 5aa is allowed to be stored temporarily in the reservoir part 8.

The wall part 7 forms a cylindrical wall annularly projecting upward. The wall part 7 includes a top end 7c at the tip thereof and has the outer peripheral surface 7a and an inner peripheral surface 7b. The wall part 7 has a tapered shape with its diameter smoothly increasing toward the lower side (the base end). A portion of the bottom part 5bb that is positioned directly below the ejection port 5ae serves as a "receiving surface α" that receives blood introduced from the blood inlet 5aa. Hence, liquid such as blood that is introduced from the blood inlet 5aa flows through the flow path 5ad and the ejection port 5ae, is ejected from the ejection port 5ae toward the receiving surface α, and is stored in the reservoir part 8. Furthermore, when the liquid level reaches the top end 7c of the wall part 7 (a liquid level A), the liquid can flow over the top end 7c and flow through the opening K down into the chamber portion 5c.

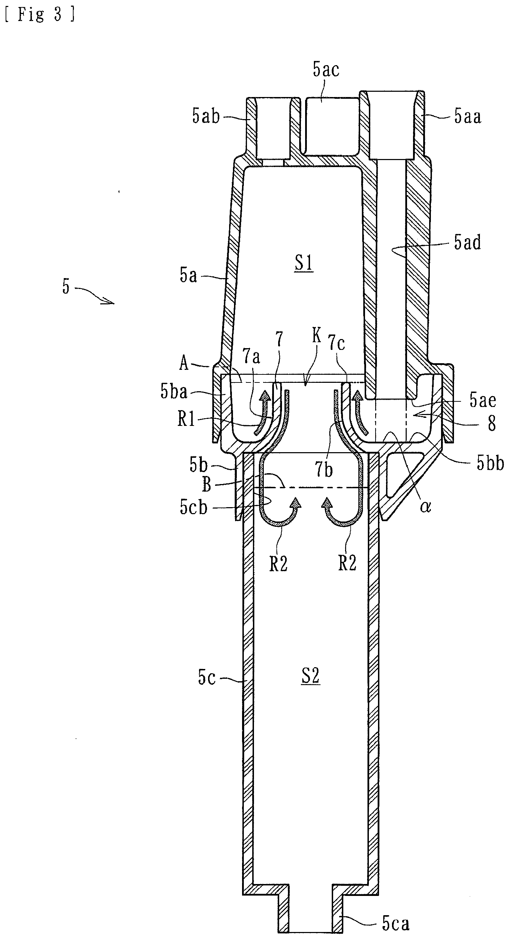

When the blood flows over the wall part 7 down into the chamber portion 5c through the opening K, the blood in the reservoir part 8 has its surface at the liquid level A, i.e., at the top end 7c of the wall part 7, whereas the blood in the storing space S2 of the chamber portion 5c has its surface at a liquid level B. In the present embodiment, as illustrated in Fig. 3, the ejection port 5ae for ejecting liquid from the flow path 5ad is positioned below the top end 7c of the wall part 7 (that is, below the liquid level A).

The wall part 7 according to the present embodiment serves as a "guiding member" that allows the blood introduced from the blood inlet 5aa to flow upward along the outer peripheral surface 7a, flow over the top end 7c, and flow down into the chamber portion 5c along the inner peripheral surface 7b. That is, when the blood ejected from the ejection port 5ae and stored in the reservoir part 8 thus overflows, the blood can flow upward, as indicated by arrows R1 illustrated in Fig. 3, while being guided along the outer peripheral surface 7a of the wall part 7. In the present embodiment, the liquid such as the blood introduced from the blood inlet 5aa and received by the receiving surface α is allowed to flow down into the chamber portion 5c along the "guiding member" (the wall part 7 in the present embodiment).

In the present embodiment, as illustrated in Fig. 3, the inner peripheral surface 7b of the wall part 7 and an inner peripheral surface 5cb of the chamber portion 5c are smoothly continuous with each other. Therefore, the liquid such as blood that has flowed over the top end 7c of the wall part 7 is allowed to flow downward along the inner peripheral surface 7b of the wall part 7 and the inner peripheral surface 5cb of the chamber portion 5c. Hence, the liquid such as blood that has flowed downward along the inner peripheral surface 7b of the wall part 7 and the inner peripheral surface 5cb of the chamber portion 5c is introduced into the liquid layer in the storing space S2 in such a manner as to swirl from the outer side toward the inner side as indicated by arrows R2 illustrated in Fig. 3.

The above embodiment employs the guiding member (the wall part 7) that allows the blood introduced from the blood inlet 5aa to flow upward and then flow downward into the chamber portion 5c. Therefore, air bubbles contained in the blood flowing upward can be easily collected into the air layer in the storing space S1. Thus, air bubbles contained in the blood can be removed to a higher extent and more assuredly. Consequently, the capacity of the chamber portion 5c can be reduced.

The present embodiment also employs the receiving surface α that receives the blood introduced from the blood inlet 5aa. Furthermore, the blood received by the receiving surface α is allowed to flow downward along the guiding member (the wall part 7) into the chamber portion 5c. Therefore, the momentum of the blood introduced from the blood inlet 5aa can be fully reduced when the blood is received by the receiving surface α. Accordingly, the foaming or the like in the chamber portion 5c can be suppressed. In particular, the guiding member according to the present embodiment is formed of the wall part 7 provided at the peripheral edge of the opening K through which blood is allowed to flow downward into the chamber portion 5c. Hence, air bubbles contained in the blood can be removed to a higher extent and more assuredly with a simple configuration. Consequently, the capacity of the chamber portion 5c can be reduced.

The wall part 7 according to the present embodiment extends over the entirety of the peripheral edge of the opening K. Furthermore, the reservoir part 8 capable of storing blood introduced from the blood inlet 5aa is provided on the side of the outer peripheral surface 7a of the wall part 7 (between the outer peripheral surface 7a and the inner peripheral surface of the outer peripheral wall 5ba). Accordingly, the blood stored in the reservoir part 8 is allowed to flow over the top end 7c of the wall part 7 and flow downward into the chamber portion 5c. Therefore, air bubbles contained in the blood can be removed while the blood flows along the guiding member (the wall part 7), and air bubbles contained in the blood can also be removed while the blood is stored in the reservoir part 8. Thus, air bubbles contained in the blood can be removed to a much higher extent and much more assuredly. Consequently, the capacity of the chamber portion 5c can further be reduced.

The inner peripheral surface 7b of the wall part 7 and the inner peripheral surface 5cb of the chamber portion 5c are continuous with each other, and the blood that has flowed over the top end 7c of the wall part 7 is allowed to flow downward along the inner peripheral surface 7b of the wall part 7 and the inner peripheral surface 5cb of the chamber portion 5c. Therefore, the foaming of the blood whose surface is formed at the liquid level B in the chamber portion 5c can be suppressed more assuredly. Furthermore, the flow path 5ad having the ejection port 5ae for ejecting the blood introduced from the blood inlet 5aa toward the reservoir part 8 is provided, and the ejection port 5ae is positioned below the top end 7c of the wall part 7. Therefore, the blood introduced from the blood inlet 5aa and ejected from the ejection port 5ae can be prevented from flowing with increased momentum toward the liquid level A of the blood stored in the reservoir part 8. Consequently, the foaming in the reservoir part 8 can be suppressed more assuredly. Hence, according to the present embodiment, a blood circuit (including an arterial blood circuit 1 and a venous blood circuit 2) provided with the above air-trap chamber 5 and a blood purification apparatus including the blood circuit can be provided.

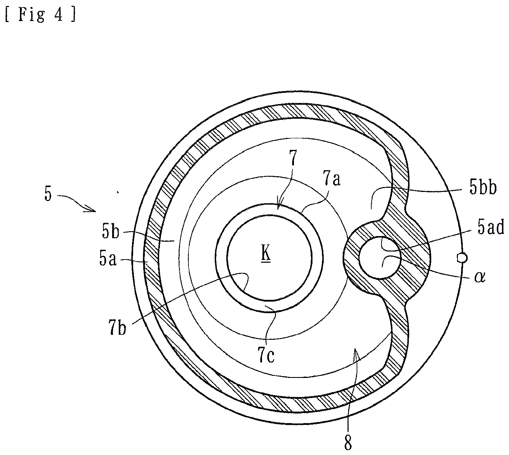

As illustrated in Fig. 4, the opening K according to the present embodiment is provided at a position decentered with respect to the middle portion 5b (a position shifted from the center of the middle portion 5b). Hence, the following advantageous effects can be produced: (a) since the radial size of the entire air-trap chamber 5 can be reduced, the air-trap chamber 5 can be manufactured in a compact size with a reduced amount of material; (b) since the air-trap chamber 5 can be manufactured in a compact size, the priming volume (the amount of liquid to be charged) can be reduced; (c) since the air-trap chamber 5 can be manufactured in a compact size, the time period (the retention time) for the substitution of the blood as the predetermined liquid in the air-trap chamber 5 can be reduced, suppressing the occurrence of blood coagulation and the generation of thrombus; and (d) since the liquid flow route (a side thereof opposite the ejection side) is narrowed gradually, the flow speed is increased and the time period (the retention time) for the substitution of the blood as the predetermined liquid in the air-trap chamber 5 can be reduced, suppressing the occurrence of blood coagulation and the generation of thrombus. While the present embodiment concerns a case where the opening K is provided at a position decentered with respect to the middle portion 5b, the opening K may be provided at the center of the middle portion 5b.

Now, a second embodiment of the present invention will be described.

As with the case of the first embodiment, an air-trap chamber according to the second embodiment is connected to a blood flow route in which blood is made to flow, and is intended for collecting and removing air bubbles contained in the blood flowing in the blood flow route. The air-trap chamber is applied to the blood purification apparatus illustrated in Fig. 1 and is connected to the venous blood circuit 2. Elements that are the same as those described in the first embodiment are denoted by respective ones of the reference numerals, and description of those elements is omitted.

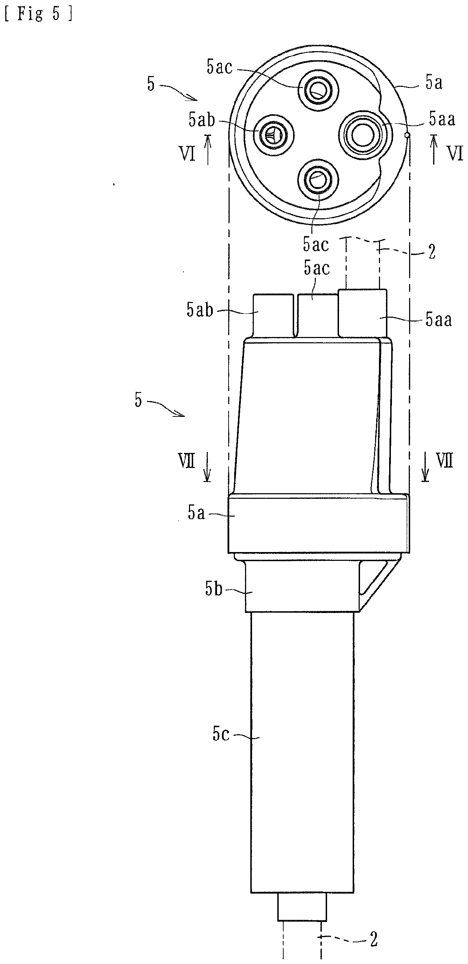

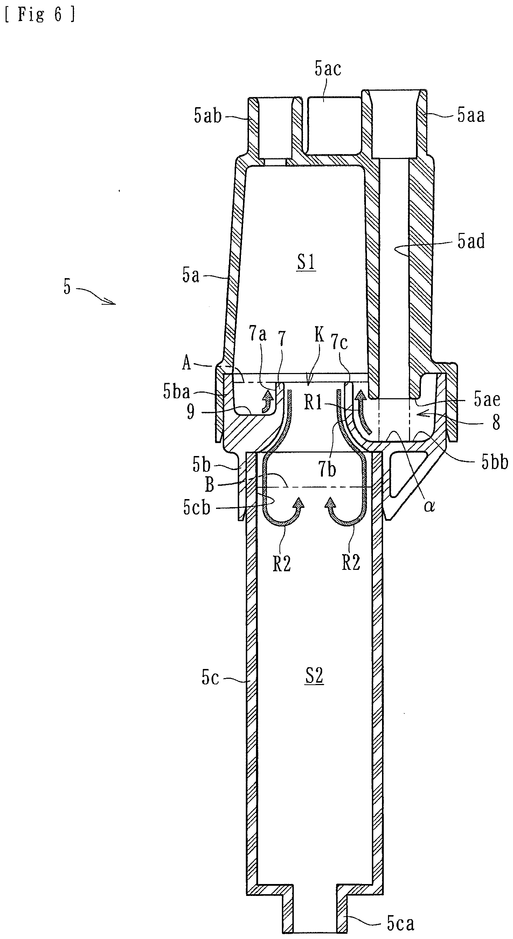

As illustrated in Figs. 5 to 9, the air-trap chamber 5 according to the second embodiment basically includes the air-layer portion 5a on the upper side, the chamber portion 5c on the lower side, and the middle portion 5b interposed between the air-layer portion 5a and the chamber portion 5c. The middle portion 5b includes the outer peripheral wall 5ba, the bottom part 5bb, the wall part 7, the reservoir part 8, and a projecting portion 9. The outer peripheral wall 5ba forms a substantially annular wall extending along the outline of the middle portion 5b. The opening K is provided on the inner side of the outer peripheral wall 5ba. The opening K corresponds to a region where the storing space S1 of the air-layer portion 5a and the storing space S2 of the chamber portion 5c communicate with each other. The wall part 7 projects from and extends over the entirety of the peripheral edge of the opening K.

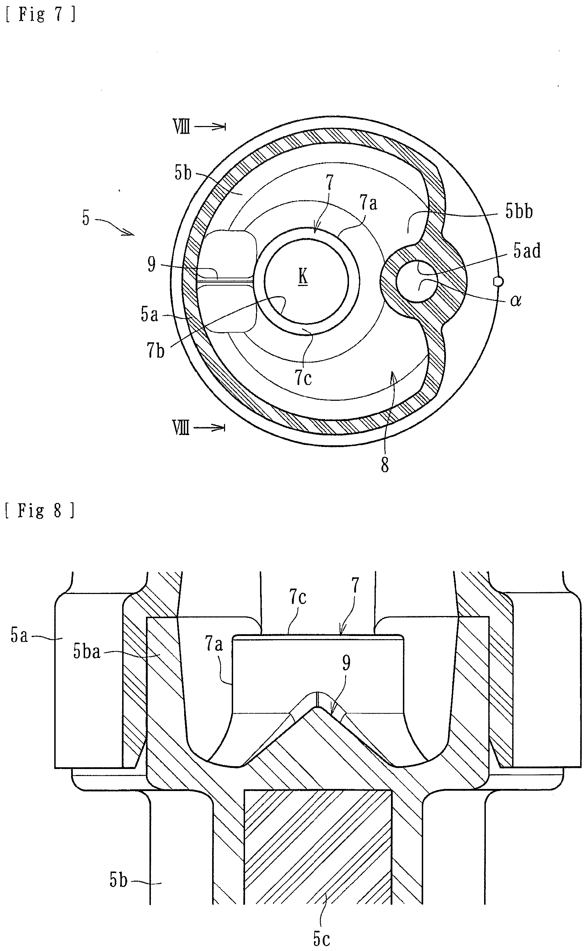

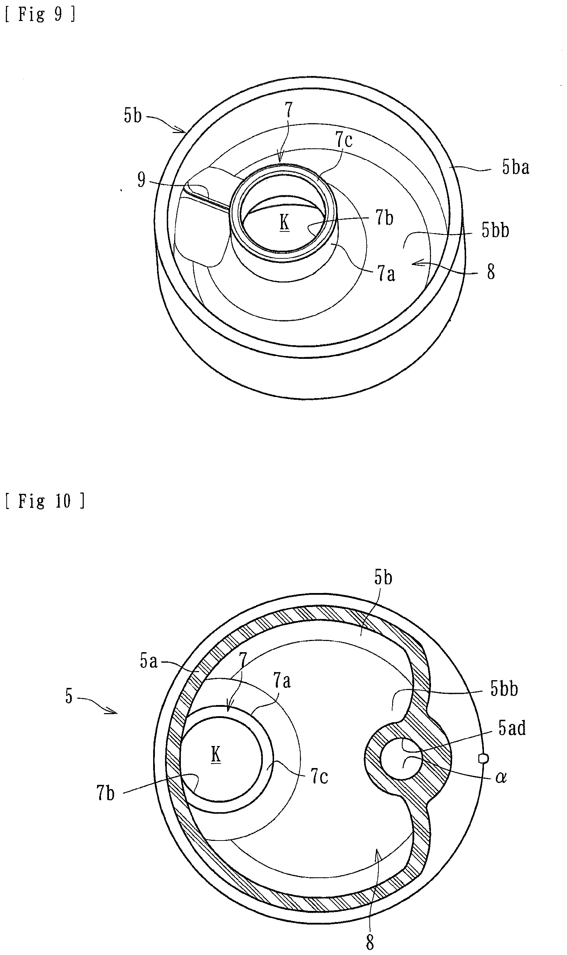

As illustrated in Figs. 7 and 9, the projecting portion 9 according to the second embodiment projects upward from the bottom part 5bb and is provided across the wall part 7 from the receiving surface α. The projecting portion 9 is intended for suppressing the retention of blood. The blood that is yet to flow over the top end 7c into the opening K particularly tends to be retained in the region across the wall part 7 from the receiving surface α. Hence, the projecting portion 9 is provided in that region. Thus, the overflow in that region can be caused more proactively, and the retention of blood can be suppressed.

To suppress the retention of blood in the region across the wall part 7 from the receiving surface α, a portion of the wall part 7 may be integrated with the outer peripheral wall 5ba of the middle portion 5b as illustrated in Fig. 10. Specifically, by integrating a portion of the wall part 7 that is opposite the receiving surface α with the outer peripheral wall 5ba, blood can be prevented from being retained in the region where blood tends to be retained (the region across the wall part 7 from the receiving surface α). Thus, the retention in that region can be suppressed assuredly.

As with the first embodiment, the second embodiment employs the guiding member (the wall part 7) that allows the blood introduced from the blood inlet 5aa to flow upward and then downward into the chamber portion 5c. Hence, the air bubbles contained in the blood can be easily collected into the air layer in the storing space S1 when the blood flows upward. Accordingly, the air bubbles contained in the blood can be removed to a higher extent and more assuredly. Consequently, the capacity of the chamber portion 5c can be reduced.

The second embodiment also employs the receiving surface α that receives the blood introduced from the blood inlet 5aa. Furthermore, the blood received by the receiving surface α is allowed to flow downward along the guiding member (the wall part 7) into the chamber portion 5c. Therefore, the momentum of the blood introduced from the blood inlet 5aa can be fully reduced when the blood is received by the receiving surface α. Accordingly, the foaming or the like in the chamber portion 5c can be suppressed. In particular, the guiding member according to the second embodiment is formed of the wall part 7 provided at the peripheral edge of the opening K through which blood is allowed to flow downward into the chamber portion 5c. Hence, air bubbles contained in the blood can be removed to a higher extent and more assuredly with a simple configuration. Consequently, the capacity of the chamber portion 5c can be reduced.

The wall part 7 according to the second embodiment extends over the entirety of the peripheral edge of the opening K. Furthermore, the reservoir part 8 capable of storing blood introduced from the blood inlet 5aa is provided on the side of the outer peripheral surface 7a of the wall part 7 (between the outer peripheral surface 7a and the inner peripheral surface of the outer peripheral wall 5ba). Accordingly, the blood stored in the reservoir part 8 is allowed to flow over the top end 7c of the wall part 7 and flow downward into the chamber portion 5c. Therefore, air bubbles contained in the blood can be removed while the blood flows along the guiding member (the wall part 7), and air bubbles contained in the blood can also be removed while the blood is stored in the reservoir part 8. Thus, air bubbles contained in the blood can be removed to a much higher extent and much more assuredly. Consequently, the capacity of the chamber portion 5c can further be reduced.

The inner peripheral surface 7b of the wall part 7 and the inner peripheral surface 5cb of the chamber portion 5c are continuous with each other, and the blood that has flowed over the top end 7c of the wall part 7 is allowed to flow downward along the inner peripheral surface 7b of the wall part 7 and the inner peripheral surface 5cb of the chamber portion 5c. Therefore, the foaming of the blood whose surface is formed at the liquid level B in the chamber portion 5c can be suppressed more assuredly. Furthermore, the flow path 5ad having the ejection port 5ae for ejecting the blood introduced from the blood inlet 5aa toward the reservoir part 8 is provided, and the ejection port 5ae is positioned below the top end 7c of the wall part 7. Therefore, the blood introduced from the blood inlet 5aa and ejected from the ejection port 5ae can be prevented from flowing with increased momentum toward the liquid level A of the blood stored in the reservoir part 8. Consequently, the foaming in the reservoir part 8 can be suppressed more assuredly. Hence, according to the second embodiment, a blood circuit (including an arterial blood circuit 1 and a venous blood circuit 2) provided with the above air-trap chamber 5 and a blood purification apparatus including the blood circuit can be provided.

As illustrated in Fig. 7, the opening K according to the second embodiment is provided at a position decentered with respect to the middle portion 5b (a position shifted from the center of the middle portion 5b). Hence, the following advantageous effects can be produced: (a) since the radial size can be reduced, the air-trap chamber 5 can be manufactured in a compact size with a reduced amount of material; (b) since the air-trap chamber 5 can be manufactured in a compact size, the priming volume (the amount of liquid to be charged) can be reduced; (c) since the air-trap chamber 5 can be manufactured in a compact size, the time period (the retention time) for the substitution of the blood as the predetermined liquid in the air-trap chamber 5 can be reduced, suppressing the occurrence of blood coagulation and the generation of thrombus; and (d) since the liquid flow route (a side thereof opposite the ejection side) is narrowed gradually, the flow speed is increased and the time period (the retention time) for the substitution of the blood as the predetermined liquid in the air-trap chamber can be reduced, suppressing the occurrence of blood coagulation and the generation of thrombus. While the second embodiment concerns a case where the opening K is provided at a position decentered with respect to the middle portion 5b, the opening K may be provided at the center of the middle portion 5b.

Now, a third embodiment of the present invention will be described.

As with the case of the first embodiment, an air-trap chamber according to the third embodiment is connected to a blood flow route in which blood is made to flow, and is intended for collecting and removing air bubbles contained in the blood flowing in the blood flow route. The air-trap chamber is applied to the blood purification apparatus illustrated in Fig. 1 and is connected to the venous blood circuit 2. Elements that are the same as those described in the first embodiment are denoted by respective ones of the reference numerals, and description of those elements is omitted.

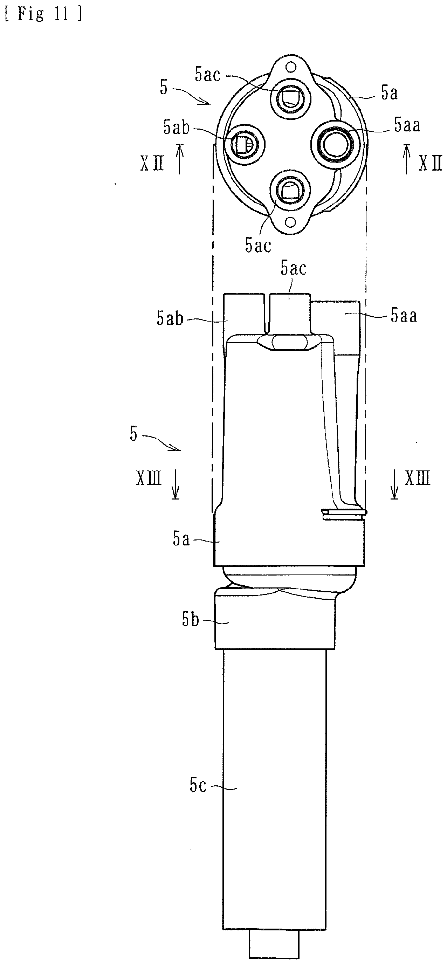

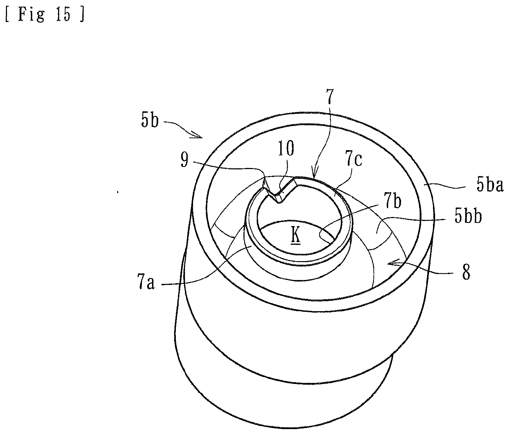

As illustrated in Figs. 11 to 15, the air-trap chamber 5 according to the third embodiment basically includes the air-layer portion 5a on the upper side, the chamber portion 5c on the lower side, and the middle portion 5b interposed between the air-layer portion 5a and the chamber portion 5c. The middle portion 5b includes the outer peripheral wall 5ba, the bottom part 5bb, the wall part 7, the reservoir part 8, and a cut part 10. The outer peripheral wall 5ba forms a substantially annular wall extending along the outline of the middle portion 5b. The opening K is provided on the inner side of the outer peripheral wall 5ba. The opening K corresponds to a region where the storing space S1 of the air-layer portion 5a and the storing space S2 of the chamber portion 5c communicate with each other. The wall part 7 projects from and extends over the entirety of the peripheral edge of the opening K.

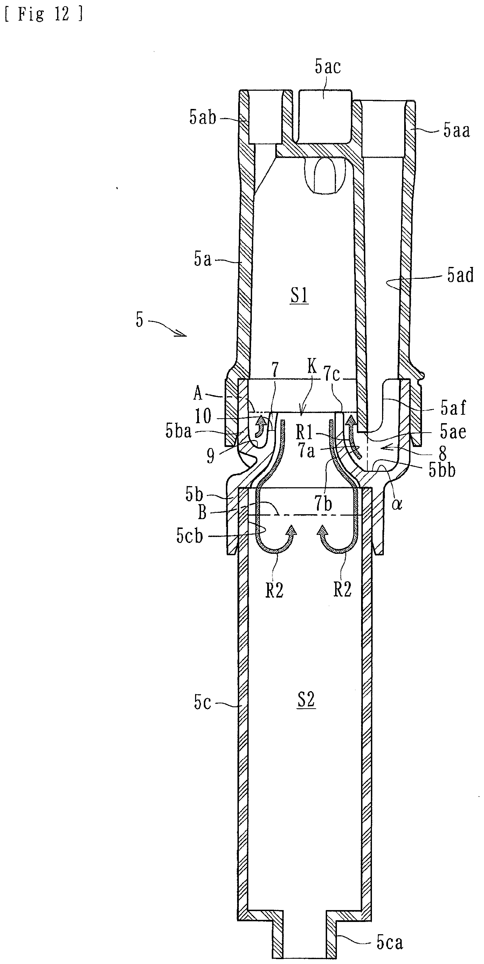

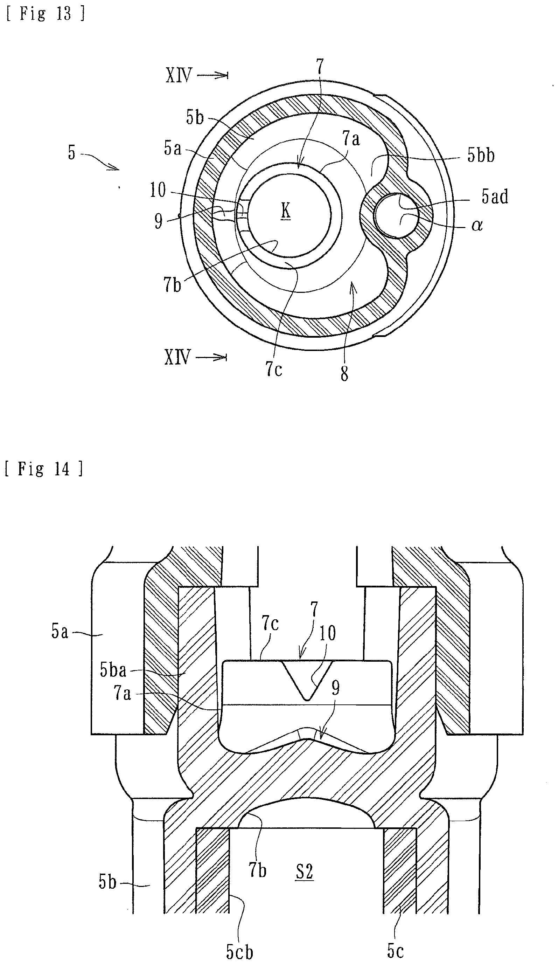

As illustrated in Figs. 12 and 13, the cut part 10 according to the third embodiment is provided by cutting a region of the wall part 7 that is on the side farthest from the receiving surface α. The cut part 10 is intended for suppressing the retention of blood. Specifically, the blood that is yet to flow over the top end 7c into the opening K particularly tends to be retained at the position across the wall part 7 from the receiving surface α. Hence, the cut part 10 is provided in that region of the wall part 7. Thus, the overflow in that region can be caused more proactively, and the retention of blood can be suppressed.

As with the first embodiment, the third embodiment employs the guiding member (the wall part 7) that allows the blood introduced from the blood inlet 5aa to flow upward and then downward into the chamber portion 5c. Hence, the air bubbles contained in the blood can be easily collected into the air layer in the storing space S1 when the blood flows upward. Accordingly, the air bubbles contained in the blood can be removed to a higher extent and more assuredly. Consequently, the capacity of the chamber portion 5c can be reduced.

The third embodiment also employs the receiving surface α that receives the blood introduced from the blood inlet 5aa. Furthermore, the blood received by the receiving surface α is allowed to flow downward along the guiding member (the wall part 7) into the chamber portion 5c. Therefore, the momentum of the blood introduced from the blood inlet 5aa can be fully reduced when the blood is received by the receiving surface α. Accordingly, the foaming or the like in the chamber portion 5c can be suppressed. In particular, the guiding member according to the third embodiment is formed of the wall part 7 provided at the peripheral edge of the opening K through which blood is allowed to flow downward into the chamber portion 5c. Hence, air bubbles contained in the blood can be removed to a higher extent and more assuredly with a simple configuration. Consequently, the capacity of the chamber portion 5c can be reduced.

The wall part 7 according to the third embodiment extends over the entirety of the peripheral edge of the opening K. Furthermore, the reservoir part 8 capable of storing blood introduced from the blood inlet 5aa is provided on the side of the outer peripheral surface 7a of the wall part 7 (between the outer peripheral surface 7a and the inner peripheral surface of the outer peripheral wall 5ba). Accordingly, the blood stored in the reservoir part 8 is allowed to flow over the top end 7c of the wall part 7 and flow downward into the chamber portion 5c. Therefore, air bubbles contained in the blood can be removed while the blood flows along the guiding member (the wall part 7), and air bubbles contained in the blood can also be removed while the blood is stored in the reservoir part 8. Thus, air bubbles contained in the blood can be removed to a much higher extent and much more assuredly. Consequently, the capacity of the chamber portion 5c can further be reduced.

The inner peripheral surface 7b of the wall part 7 and the inner peripheral surface 5cb of the chamber portion 5c are continuous with each other, and the blood that has flowed over the top end 7c of the wall part 7 is allowed to flow downward along the inner peripheral surface 7b of the wall part 7 and the inner peripheral surface 5cb of the chamber portion 5c. Therefore, the foaming of the blood whose surface is formed at the liquid level B in the chamber portion 5c can be suppressed more assuredly. Furthermore, the flow path 5ad having the ejection port 5ae for ejecting the blood introduced from the blood inlet 5aa toward the reservoir part 8 is provided, and the ejection port 5ae is positioned below the top end 7c of the wall part 7. Therefore, the blood introduced from the blood inlet 5aa and ejected from the ejection port 5ae can be prevented from flowing with increased momentum toward the liquid level A of the blood stored in the reservoir part 8. Consequently, the foaming in the reservoir part 8 can be suppressed more assuredly. Hence, according to the third embodiment, a blood circuit (including an arterial blood circuit 1 and a venous blood circuit 2) provided with the above air-trap chamber 5 and a blood purification apparatus including the blood circuit can be provided.

As illustrated in Fig. 13, the opening K according to the third embodiment is provided at a position decentered with respect to the middle portion 5b (a position shifted from the center of the middle portion 5b). Hence, the following advantageous effects can be produced: (a) since the radial size can be reduced, the air-trap chamber 5 can be manufactured in a compact size with a reduced amount of material; (b) since the air-trap chamber 5 can be manufactured in a compact size, the priming volume (the amount of liquid to be charged) can be reduced; (c) since the air-trap chamber 5 can be manufactured in a compact size, the time period (the retention time) for the substitution of the blood as the predetermined liquid in the air-trap chamber 5 can be reduced, suppressing the occurrence of blood coagulation and the generation of thrombus; and (d) since the liquid flow route (a side thereof opposite the ejection side) is narrowed gradually, the flow speed is increased and the time period (the retention time) for the substitution of the blood as the predetermined liquid in the air-trap chamber can be reduced, suppressing the occurrence of blood coagulation and the generation of thrombus. While the third embodiment concerns a case where the opening K is provided at a position decentered with respect to the middle portion 5b, the opening K may be provided at the center of the middle portion 5b.

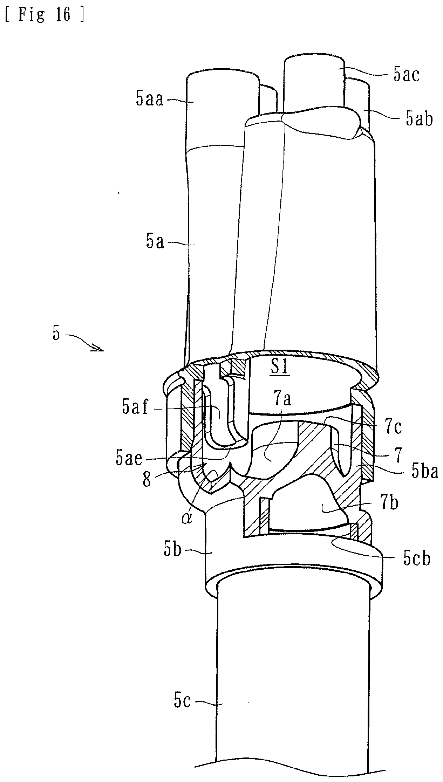

Furthermore, as illustrated in Fig. 16, the ejection port 5ae according to the third embodiment a cut 5af. Hence, the following advantageous effects can be produced: (a) since the radial size can be reduced, the air-trap chamber 5 can be manufactured in a compact size with a reduced amount of material; (b) since the air-trap chamber 5 can be manufactured in a compact size, the priming volume (the amount of liquid to be charged) can be reduced; (c) since the air-trap chamber 5 can be manufactured in a compact size, the time period (the retention time) for the substitution of the blood as the predetermined liquid in the air-trap chamber 5 can be reduced, suppressing the occurrence of blood coagulation and the generation of thrombus; and (d) since the liquid flow route (a side thereof opposite the ejection side) is narrowed gradually, the flow speed is increased and the time period (the retention time) for the substitution of the blood as the predetermined liquid in the air-trap chamber can be reduced, suppressing the occurrence of blood coagulation and the generation of thrombus.

Now, a fourth embodiment of the present invention will be described.

As with the case of the first embodiment, an air-trap chamber according to the fourth embodiment is connected to a blood flow route in which blood is made to flow, and is intended for collecting and removing air bubbles contained in the blood flowing in the blood flow route. The air-trap chamber is applied to the blood purification apparatus illustrated in Fig. 1 and is connected to the venous blood circuit 2. Elements that are the same as those described in the first embodiment are denoted by respective ones of the reference numerals, and description of those elements is omitted.

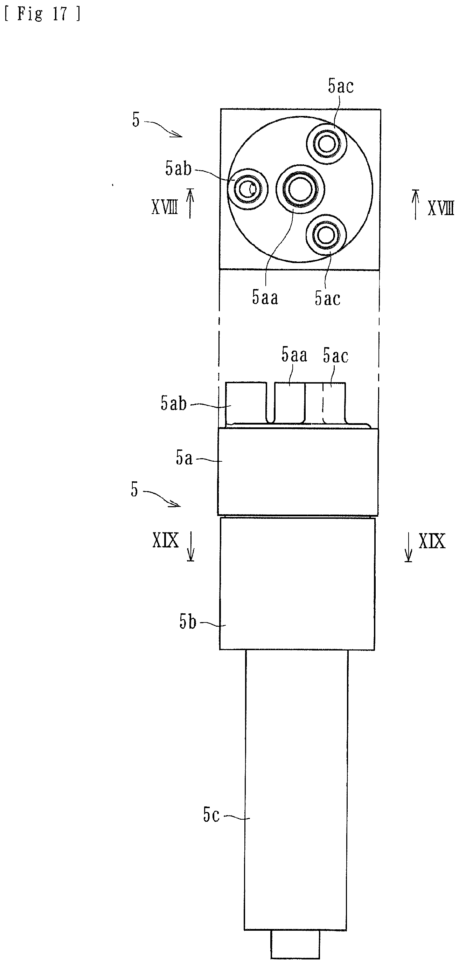

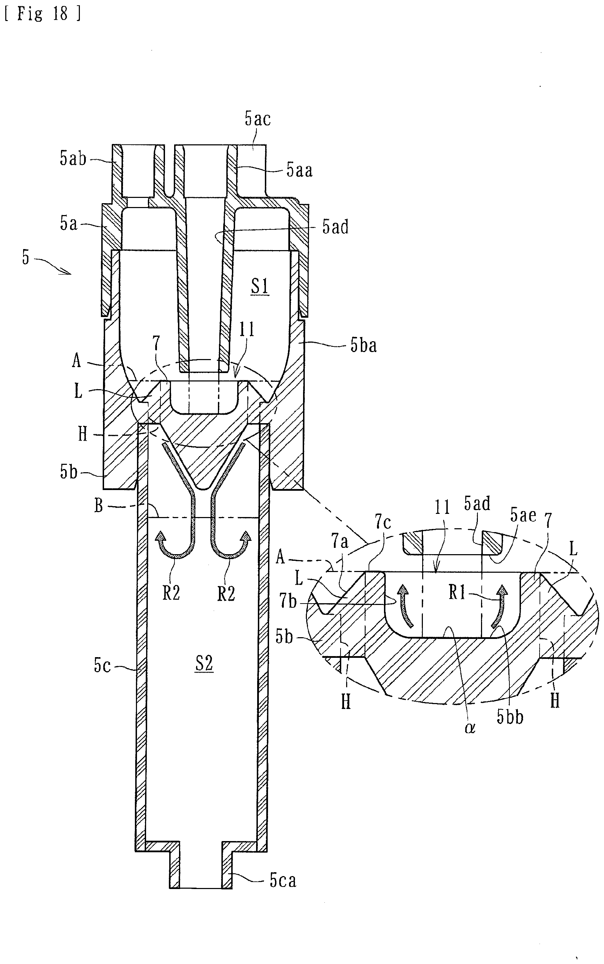

As illustrated in Figs. 17 to 20, the air-trap chamber 5 according to the fourth embodiment basically includes the air-layer portion 5a on the upper side, the chamber portion 5c on the lower side, and the middle portion 5b interposed between the air-layer portion 5a and the chamber portion 5c. The middle portion 5b includes the outer peripheral wall 5ba, the bottom part 5bb, the wall part 7, and a reservoir part 11. The reservoir part 11 according to the fourth embodiment is provided on the inner side of the wall part 7 (the guiding member). The receiving surface α that receives the blood introduced from the blood inlet 5aa is formed at the bottom of the reservoir part 11.

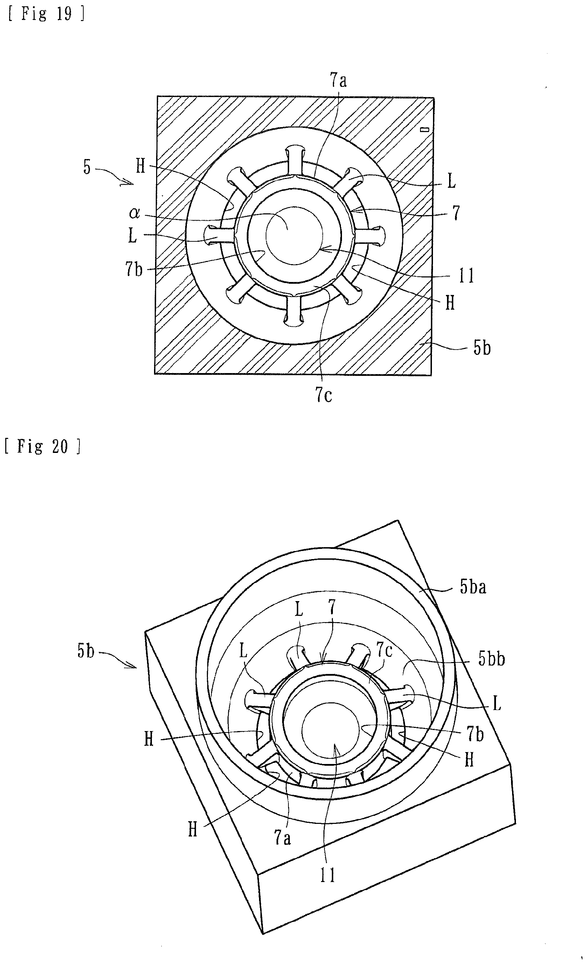

Furthermore, as illustrated in Fig. 19, the wall part 7 providing the reservoir part 11 on the inner side thereof is connected to the outer peripheral wall 5ba of the middle portion 5b with a plurality of ribs L, and openings H are each provided between adjacent ones of the ribs L. The openings H corresponds to a region where the storing space S1 of the air-layer portion 5a and the storing space S2 of the chamber portion 5c communicate with each other. The liquid such as the blood introduced from the blood inlet 5aa and flowed through the flow path 5ad is ejected from the ejection port 5ae toward the receiving surface α and is stored in the reservoir part 11. When the liquid level reaches the top end 7c (the liquid level A) of the wall part 7, the liquid flows over the top end 7c and runs down into the chamber portion 5c through the openings H.

The fourth embodiment employs the guiding member (the wall part 7) that allows the blood introduced from the blood inlet 5aa to flow upward and then flow downward into the chamber portion 5c. Hence, the air bubbles contained in the blood can be easily collected into the air layer in the storing space S1 when the blood flows upward. Accordingly, the air bubbles contained in the blood can be removed to a higher extent and more assuredly. Consequently, the capacity of the chamber portion 5c can be reduced.

The fourth embodiment also employs the receiving surface α that receives the blood introduced from the blood inlet 5aa. Furthermore, the blood received by the receiving surface α is allowed to flow downward along the guiding member (the wall part 7) into the chamber portion 5c. Therefore, the momentum of the blood introduced from the blood inlet 5aa can be fully reduced when the blood is received by the receiving surface α. Accordingly, the foaming or the like in the chamber portion 5c can be suppressed. Furthermore, the fourth embodiment employs the flow path 5ad having the ejection port 5ae for ejecting the blood introduced from the blood inlet 5aa toward the reservoir part 11, and the ejection port 5ae is positioned below the top end 7c of the wall part 7. Therefore, the blood introduced from the blood inlet 5aa and ejected from the ejection port 5ae can be prevented from flowing with increased momentum toward the liquid level A of the blood stored in the reservoir part 11. Consequently, the foaming in the reservoir part 11 can be suppressed more assuredly. Hence, according to the fourth embodiment, a blood circuit (including an arterial blood circuit 1 and a venous blood circuit 2) provided with the above air-trap chamber 5 and a blood purification apparatus including the blood circuit can be provided.

While some embodiments have been described above, the present invention is not limited thereto. For example, the guiding member that allows the blood introduced from the blood inlet 5aa to flow upward and then to flow downward into the chamber portion 5c may be another element different from the wall part 7. While the above embodiments each concern a case where the air-layer portion 5a, the middle portion 5b, and the chamber portion 5c are separate components that are assembled into the air-trap chamber 5, the air-layer portion 5a, the middle portion 5b, and the chamber portion 5c may alternatively be provided as one integrated component. While the above embodiments each concern a case where the air-trap chamber 5 is connected to the venous blood circuit 2, the air-trap chamber 5 may also be applied to an air-trap chamber connected to the arterial blood circuit 1 or another blood flow route in which blood is made to flow. While the above embodiments each concern a case where blood is introduced into and discharged from the chamber portion, any other predetermined liquid, such as a physiological saline solution, a drug solution, or the like, may be introduced into and discharged from the chamber portion.

### Industrial Applicability

The present invention is also applicable to any other air-trap chamber having, for example, a different external shape or other additional functions, as long as the air-trap chamber includes a guiding member that allows a predetermined liquid introduced from a liquid inlet to flow upward and then to flow downward into a chamber portion.

### Reference Signs List

- 1: arterial blood circuit (liquid flow route)
- 2: venous blood circuit (liquid flow route)
- 3: dialyzer (blood purifier)
- 4: blood pump
- 5: air-trap chamber
- 6: container unit
- 7: wall part
- 7a: outer peripheral surface
- 7b: inner peripheral surface
- 7c: top end
- 8: reservoir part
- 9: projecting portion
- 10: cut part
- 11: reservoir part
- K: opening
- H: opening

## Claims

1. An air-trap chamber (5) connected to a liquid flow route in which a predetermined liquid is made to flow, the air-trap chamber (5) including a chamber portion (5c) capable of storing the predetermined liquid by a predetermined amount, a liquid inlet (5aa) from which the predetermined liquid in the liquid flow route is allowed to be introduced into the chamber portion (5c), and a liquid outlet (5ca) from which the predetermined liquid in the chamber portion (5c) is allowed to be discharged into the liquid flow route, the air-trap chamber (5) being configured to collect and remove air bubbles contained in the predetermined liquid stored in the chamber portion (5c), the air-trap chamber (5) comprising:
a guiding member that allows the predetermined liquid introduced from the liquid inlet (5aa) to flow upward and then to flow downward into the chamber portion (5c), wherein the guiding member is a wall part (7) formed at a peripheral edge of an opening (K) that allows the predetermined liquid to flow downward into the chamber portion (5c), and an air-layer portion (5a) on an upper side and a middle portion (5b) interposed between the air-layer portion (5a) and the chamber portion (5c),
the air-trap chamber (5) being **characterized in that** the opening (K) is provided in the middle portion (5b) and is provided at a position decentered with respect to the middle portion (5b), so that the liquid flow route at a side opposite the side where the predetermined liquid is introduced from the liquid inlet (5aa) is narrowed gradually.

2. The air-trap chamber (5) according to Claim 1, **characterized in** further comprising a receiving surface (α) that receives the predetermined liquid introduced from the liquid inlet (5aa) and **in that** the predetermined liquid received by the receiving surface (α) is allowed to flow downward along the guiding member into the chamber portion (5c).

3. The air-trap chamber (5) according to Claim 1 or 2, **characterized in that** the wall part (7) extends over an entirety of a peripheral edge of the opening (K) and has, on an outer-peripheral side of the wall part (7), a reservoir part (8) capable of storing the predetermined liquid introduced from the liquid inlet (5aa), and **in that** the predetermined liquid stored in the reservoir part (8) is allowed to flow over a top end (7c) of the wall part (7) and to flow downward into the chamber portion (5c).

4. The air-trap chamber (5) according to Claim 3, **characterized in** further comprising a projecting portion (9) that projects upward from a position across the wall part (7) from the receiving surface (α) that receives the predetermined liquid introduced from the liquid inlet (5aa).

5. The air-trap chamber (5) according to Claim 3, **characterized in** further comprising a cut part (10) provided by cutting a region across the wall part (7) from the receiving surface (α) that receives the predetermined liquid introduced from the liquid inlet (5aa).

6. The air-trap chamber (5) according to any of Claims 3 to 5, **characterized in** further comprising: an ejection port (5ae) that ejects, toward the reservoir part (8), the predetermined liquid introduced from the liquid inlet (5aa); and a cut (5af) provided in the ejection port (5ae).

7. The air-trap chamber (5) according to any of Claims 3 to 6, **characterized in that** an inner peripheral surface (7b) of the wall part (7) and an inner peripheral surface (5cb) of the chamber portion (5c) are continuous with each other, and **in that** the predetermined liquid that has flowed over the top end (7c) of the wall part (7) is allowed to flow downward along the inner peripheral surface (7b) of the wall part (7) and the inner peripheral surface (5cb) of the chamber portion (5c).

8. The air-trap chamber (5) according to any of Claims 3 to 7, **characterized in** further comprising a flow path (5ad) having an ejection port (5ae) from which the predetermined liquid introduced from the liquid inlet (5aa) is ejected toward the reservoir part (8), and **in that** the ejection port (5ae) is positioned below the top end (7c) of the wall part (7).

9. A blood circuit **characterized in** comprising the air-trap chamber (5) according to any of Claims 1 to 8.

10. A blood purification apparatus **characterized in** comprising the blood circuit according to Claim 9.

11. Use of the air-trap chamber (5) according to any one of Claims 1 to 8, **characterized in that** the predetermined liquid forms a liquid level (B) in the chamber portion (5c).

## Patentansprüche

1. Luftabscheidekammer (5), die mit einem Flüssigkeitsströmungsweg verbunden ist, in dem eine vorbestimmte Flüssigkeit zum Strömen gebracht wird, wobei die Luftabscheidekammer (5) einen Kammerabschnitt (5c), der in der Lage ist, die vorbestimmte Flüssigkeit um eine vorbestimmte Menge zu speichern, einen Flüssigkeitseinlaß (5aa), von dem die vorbestimmte Flüssigkeit in den Flüssigkeitsströmungsweg in den Kammerabschnitt (5c) eingeführt werden kann und einen Flüssigkeitsauslass (5ca), von dem die vorbestimmte Flüssigkeit in dem Kammerabschnitt (5c) in den Flüssigkeitsströmungsweg abgegeben werden kann, wobei die Luftabscheidekammer (5) so konfiguriert ist, dass sie Luftblasen, die in der in dem Kammerabschnitt (5c) gespeicherten vorbestimmten Flüssigkeit enthalten sind, sammelt und entfernt, wobei die Luftabscheidekammer (5) umfasst
ein Leitelement, das es der vom Flüssigkeitseinlass (5aa) eingeführten vorbestimmten Flüssigkeit ermöglicht, nach oben und dann nach unten in den Kammerabschnitt (5c) zu fließen, wobei das Leitelement ein Wandteil (7) ist, das an einem Umfangsrand einer Öffnung (K) ausgebildet ist, die es der vorbestimmten Flüssigkeit ermöglicht, nach unten in den Kammerabschnitt (5c) zu fließen, und
einen Luftschichtabschnitt (5a) an einer Oberseite und einen Mittelabschnitt (5b), der zwischen dem Luftschichtabschnitt (5a) und dem Kammerabschnitt (5c) angeordnet ist,
wobei die Luftabscheidekammer (5) **dadurch gekennzeichnet ist, dass** die Öffnung (K) in dem mittleren Abschnitt (5b) vorgesehen ist und an einer Position vorgesehen ist, die in Bezug auf den mittleren Abschnitt (5b) dezentriert ist, so dass der Flüssigkeitsströmungsweg an einer Seite gegenüber der Seite, an der die vorbestimmte Flüssigkeit von dem Flüssigkeitseinlass (5aa) eingeführt wird, allmählich verengt wird.

2. Luftabscheidekammer (5) nach Anspruch 1, **dadurch gekennzeichnet, dass** sie ferner eine Aufnahmefläche () umfasst, die die vom Flüssigkeitseinlass (5aa) eingeleitete vorbestimmte Flüssigkeit aufnimmt, und dass die von der Aufnahmefläche () aufgenommene vorbestimmte Flüssigkeit entlang des Führungselements nach unten in den Kammerabschnitt (5c) fließen kann.

3. Luftabscheidekammer (5) nach Anspruch 1 oder 2, **dadurch gekennzeichnet, daß** sich das Wandteil (7) über die Gesamtheit eines Umfangsrandes der Öffnung (K) erstreckt und an einer Außenumfangsseite des Wandteils (7) ein Reservoirteil (8) aufweist, das in der Lage ist, die von dem Flüssigkeitseinlaß (5aa) eingeleitete vorbestimmte Flüssigkeit zu speichern, und daß die in dem Reservoirteil (8) gespeicherte vorbestimmte Flüssigkeit über ein oberes Ende (7c) des Wandteils (7) fließen und nach unten in den Kammerabschnitt (5c) fließen kann.

4. Luftabscheidekammer (5) nach Anspruch 3, **dadurch gekennzeichnet, dass** sie ferner einen vorstehenden Abschnitt (9) umfasst, der von einer Position quer zum Wandteil (7) von der Aufnahmefläche (), die die vom Flüssigkeitseinlass (5aa) eingeführte vorbestimmte Flüssigkeit aufnimmt, nach oben ragt.

5. Luftabscheidekammer (5) nach Anspruch 3, **dadurch gekennzeichnet, dass** sie ferner einen abgeschnittenen Teil (10) umfasst, der durch Schneiden eines Bereichs quer zum Wandteil (7) von der Aufnahmefläche (), die die vom Flüssigkeitseinlass (5aa) eingeführte vorbestimmte Flüssigkeit aufnimmt, gebildet ist.

6. Luftabscheidekammer (5) nach einem der Ansprüche 3 bis 5, **dadurch gekennzeichnet, dass** sie ferner umfasst: eine Ausstoßöffnung (5ae), die die von dem Flüssigkeitseinlass (5aa) eingeleitete vorbestimmte Flüssigkeit in Richtung des Reservoirteils (8) ausstößt; und einen Schnitt (5af), der in der Ausstoßöffnung (5ae) vorgesehen ist.

7. Luftabscheidekammer (5) nach einem der Ansprüche 3 bis 6, **dadurch gekennzeichnet, dass** eine innere Umfangsfläche (7b) des Wandteils (7) und eine innere Umfangsfläche (5cb) des Kammerabschnitts (5c) kontinuierlich miteinander verbunden sind, und dass die vorbestimmte Flüssigkeit, die über das obere Ende (7c) des Wandteils (7) geflossen ist, entlang der inneren Umfangsfläche (7b) des Wandteils (7) und der inneren Umfangsfläche (5cb) des Kammerabschnitts (5c) nach unten fließen kann.

8. Luftabscheidekammer (5) nach einem der Ansprüche 3 bis 7, **dadurch gekennzeichnet, dass** sie ferner einen Strömungsweg (5ad) mit einer Ausstoßöffnung (5ae) umfasst, aus der die vom Flüssigkeitseinlass (5aa) eingeführte vorbestimmte Flüssigkeit in Richtung des Reservoirteils (8) ausgestoßen wird, und dass die Ausstoßöffnung (5ae) unterhalb des oberen Endes (7c) des Wandteils (7) angeordnet ist.

9. Blutkreislauf, **dadurch gekennzeichnet, dass** er die Luftabscheidekammer (5) nach einem der Ansprüche 1 bis 8 umfasst.

10. Blutreinigungsvorrichtung, **dadurch gekennzeichnet, dass** sie den Blutkreislauf nach Anspruch 9 umfasst.

11. Verwendung der Luftabscheidekammer (5) nach einem der Ansprüche 1 bis 8, **dadurch gekennzeichnet, dass** die vorbestimmte Flüssigkeit einen Flüssigkeitsspiegel (B) im Kammerteil (5c) bildet.

## Revendications

1. Chambre de piège à air (5) connectée à une voie d'écoulement de liquide dans laquelle un liquide prédéterminé est fait circuler, ladite chambre de piège à air (5) comprenant une partie de chambre (5c) apte à stocker du liquide prédéterminé d'une quantité prédéterminée, une entrée de liquide (5aa) par laquelle le liquide prédéterminé dans la voie d'écoulement de liquide est permis d'être introduit dans ladite partie de chambre (5c), et une sortie de liquide (5ca) par laquelle le liquide prédéterminé dans ladite partie de chambre (5c) est permis d'être déchargé dans la voie d'écoulement de liquide, ladite chambre de piège à air (5) étant configurée pour ramasser et éliminer des bulles d'air contenues dans le liquide prédéterminé stocké dans ladite partie de chambre (5c), ladite chambre de piège à air (5) comprenant :
un élément de guidage permettant que le liquide prédéterminé introduit par ladite entrée de liquide (5aa) s'écoule vers le haut et ensuite s'écoule vers le bas et dans ladite partie de chambre (5c), ledit élément de guidage étant une partie de paroi (7) formée sur un bord périphérique d'une ouverture (K) qui permet que le liquide prédéterminé s'écoule vers le bas et dans ladite partie de chambre (5c), et une partie de couche d'air (5a) sur le côté supérieur et une partie intermédiaire (5b) interposée entre ladite partie de couche d'air (5a) et ladite partie de chambre (5c),
ladite chambre de piège à air (5) étant **caractérisée en ce que** ladite ouverture (K) est prévue dans ladite partie intermédiaire (5b) et est prévue dans une position décentrée par rapport à ladite partie intermédiaire (5b) de façon que la voie d'écoulement de liquide, à un côté opposé au côté où du liquide prédéterminé est introduit par ladite entrée de liquide (5aa), se rétrécit graduellement.

2. Chambre de piège à air (5) selon la revendication 1, **caractérisée en ce qu'**elle comprend en outre une surface de réception (α) recevant le liquide prédéterminé introduit par ladite entrée de liquide (5aa), et **en ce que** le liquide prédéterminé reçu par ladite surface de réception (α) est permis de s'écouler vers le bas le long dudit élément de guidage et dans ladite partie de chambre (5c).

3. Chambre de piège à air (5) selon la revendication 1 ou 2, **caractérisée en ce que** ladite partie de paroi (7) s'étend sur une totalité d'un bord périphérique de ladite ouverture (K) et comprend, sur une face périphérique extérieure de ladite partie de paroi (7), une partie de réservoir (8) apte à stocker le liquide prédéterminé introduit par ladite entrée de liquide (5aa), et **en ce que** le liquide prédéterminé stocké dans la partie de réservoir (8) est permis de s'écouler sur une extrémité supérieure (7c) de ladite partie de paroi (7) et à s'écouler vers le bas et dans ladite partie de chambre (5c).

4. Chambre de piège à air (5) selon la revendication 3, **caractérisée en ce qu'**elle comprend en outre une partie saillante (9) qui est en saillie vers le haut depuis une position de l'autre côté de ladite partie de paroi (7) à partir de ladite surface de réception (α) recevant le liquide prédéterminé introduit par ladite entrée de liquide (5aa).

5. Chambre de piège à air (5) selon la revendication 3, **caractérisée en ce qu'**elle comprend en outre une partie coupée (10) produit en coupant une région de l'autre côté de ladite partie de paroi (7) à partir de ladite surface de réception (α) recevant le liquide prédéterminé introduit par ladite entrée de liquide (5aa).

6. Chambre de piège à air (5) selon l'une quelconque des revendications 3 à 5, **caractérisée en ce qu'**elle comprend en outre : un orifice d'éjection (5ae) éjectant, vers ladite partie de réservoir (8), le liquide prédéterminé introduit par ladite entrée de liquide (5aa) ; et une incision (5af) prévue dans ledit orifice d'éjection (5ae).

7. Chambre de piège à air (5) selon l'une quelconque des revendications 3 à 6, **caractérisée en ce qu'**une surface périphérique intérieure (7b) de ladite partie de paroi (7) et une surface périphérique intérieure (5cb) de ladite partie de chambre (5c) sont réalisées de façon continue entre eux, et **en ce que** le liquide prédéterminé qui s'est écoulé sur l'extrémité supérieure (7c) de ladite partie de paroi (7) est permis de s'écouler vers le bas le long de la surface périphérique intérieure (7b) de ladite partie de paroi (7) et la surface périphérique intérieure (5cb) de ladite partie de chambre (5c).

8. Chambre de piège à air (5) selon l'une quelconque des revendications 3 à 7, **caractérisée en ce qu'**elle comprend en outre une voie d'écoulement (5ad) comprenant un orifice d'éjection (5ae) par lequel le liquide prédéterminé introduit par ladite entrée de liquide (5aa) est éjecté vers ladite partie de réservoir (8), et **en ce que** ledit orifice d'éjection (5ae) est positionné en dessous de l'extrémité supérieure (7c) de ladite partie de paroi (7).

9. Circuit sanguin **caractérisé en ce qu'**il comprend ledit chambre de piège à air (5) selon l'une quelconque des revendications 1 à 8.

10. Appareil de purification de sang **caractérisé en ce qu'**il comprend le circuit sanguin selon la revendication 9.

11. Utilisation de la chambre de piège à air (5) selon l'une quelconque des revendications 1 à 8, **caractérisé en ce que** le liquide prédéterminé constitue un niveau de liquide (B) dans ladite partie de chambre (5c).
